# EUROPEAN PATENT APPLICATION

(11) **EP 4 520 275 A1**
(43) Date of publication of application: **12.03.2025**
(21) Application number: 24198755.1
(22) Date of filing: 05.09.2024
(51) Int. Cl.: A61B 17/16, A61B 17/92

(54) **HANDLE ADAPTER FOR AN ORTHOPEDIC CUTTING TOOL**

(30) Priority: 06.09.2023 US 202363536775 P; 02.08.2024 US 202418793491
(71) Applicant: Zimmer, Inc., Warsaw, IN 46580 (US)
(72) Inventor: CLEMENTS, Joshua, Roanoke, 46783 (US); VANDEWALLE, Daniel, Warsaw, 46582 (US)
(74) Representative: Taor, Simon Edward William

(57) **Abstract**

As described and shown, a handle adapter for manipulating an orthopedic tool optionally including a frame optionally having a receptacle, a first slot, a second slot a coupling assembly and an actuation assembly. The frame having a proximal end portion and a distal end portion. The distal end portion optionally including the receptacle, the first slot, the second slot, the coupling assembly and the actuation assembly. The receptacle configured to receive a trunnion of the orthopedic tool. The first slot communicating with the receptacle. The first slot can be partially formed by a first ramp and a second ramp. The second slot spaced from the first slot. The coupling assembly received by the first slot and the second slot. The coupling assembly translates within the first slot and the second slot. The actuation assembly coupled to the coupling assembly and configured to drive the coupling assembly into engagement with the trunnion.

## Description

### TECHNICAL FIELD

This patent document pertains generally to orthopedic tools. More particularly, the present patent document relates to a handle adapter for use with orthopedic tools.

### BACKGROUND

A variety of surgical orthopedic tools are used to cut or otherwise shape a portion of a bone during orthopedic surgical procedures. Such orthopedic cutting tools can include broaches, reamers, rasps and the like. Such cutting tools are typically manipulated by a surgeon using a handle. The handle can be integral to or separately detachable from the cutting tool. The handle can be used for grasping the surgical cutting tool during use and to provide leverage to perform the cutting or shaping of a bone during the orthopedic surgical procedure.

In certain orthopedic procedures, it can be necessary to drill or ream an intramedullary canal of a bone. For example, in the replacement of a hip joint, it can be necessary to replace a natural femoral head with a prosthetic stem affixed within the canal of the femur. The procedure for implanting the prosthetic stem can include the use of a broach or rasp in preparing the femoral shaft for reception of the prosthetic stem by providing contouring of the femoral shaft to the gross geometry of the prosthetic stem, thereby assuring accurate location and good fit. In order to facilitate utilization of such a broach or rasp tool, it can be useful to selectively detach the working portion of the tool from the handle to facilitate precise gauging of the location of the tool within the femoral shaft, to facilitate cleaning of the tool, and for other reasons. Additionally, recently powered drivers have been developed for driving cutting of the bone using the cutting tool. These powered drivers are coupled to the handle.

A number of detachable handles for use with cutting tools, such as broaches, reamers or rasps, exist. However, these are in need of improvement for various reasons.

### OVERVIEW

Various handles known for use in connection with orthopedic cutting tools including those designed by the Applicant. Examples include, U.S. Patent Nos. 10,667,798 and 11,602,359, the disclosures of each of which is incorporated herein by reference in its entirety. The present inventors recognize, among other things, that it would be useful to have a selectively attachable handle adapter that couples to an orthopedic cutting tool in a secure manner. Furthermore, it would be useful to have the handle adapter disengage readily after use. Known handle adapters although generally effective can be improved. For example, known handle adapters have one or more drawbacks including any of: inadequate locking resulting potentially in undesired disengagement from the orthopedic cutting tool during use, loose connection to the orthopedic cutting tool resulting in excessive wear, poor kinematics that lead to loading of the linkage components that are unable to support the extraction forces, inability of the handle adapter to accommodate different coupling features, undesired point loading, etc. These drawbacks can result in undesired wear, component failure, undesired coupling, complete disengagement and other undesirable results.

With these drawbacks in mind, the present inventors have developed handle adapters that have simplified kinematics for coupling and decoupling. These simplified kinematics can include the use of a linear translation motion of a coupling assembly that couples the handle adapter to the cutting tool as compared with rotational engagement, which is commonly utilized. The handle adapters disclosed herein additionally benefit from designs that ground extraction forces to a desired component, such as a frame, without passing though linkage components. The extraction forces are also grounded to multiple locations on the frame rather than being born by a single location and/or by undesired components such as a spring of an actuation assembly. The handle adapters disclosed herein can also accommodate different trunnion designs with minimal number of components or features changed.

The present inventors have also developed the handle adapters to have a passive locking system, which allows for easy stop for the lever, reduces vibration of the lever and also allows for a spring force to keep the lever closed and/or the coupling assembly engaged to the cutting tool.

The following, non-limiting examples, detail certain aspects of the present subject matter to solve the challenges and provide the benefits discussed herein, among others.

Example 1 is a handle adapter for manipulating an orthopedic tool, optionally comprising: a frame and having a proximal end portion and a distal end portion, the distal end portion including: a receptacle configured to receive a trunnion of the orthopedic tool; a first slot communicating with the receptacle, wherein the first slot is partially formed by a first ramp and a second ramp; and a second slot spaced from the first slot; a coupling assembly received by the first slot and the second slot, wherein the coupling assembly translates within the first slot and the second slot; and an actuation assembly coupled to the coupling assembly and configured to drive the coupling assembly into engagement with the trunnion.

In Example 2, the subject matter of Example 1 optionally includes, wherein the actuation assembly includes a spring coupled to a lever, and wherein the spring is coupled to and drives the coupling assembly into the engagement with the trunnion.

In Example 3, the subject matter of Example 2 includes, wherein the spring is subject to a compressive load when the coupling assembly is in the engagement with the trunnion.

In Example 4, the subject matter of Examples 2-3 optionally includes, a locking mechanism that locks the handle in a closed position relative to the frame, wherein the locking mechanism includes a spring formed by the frame.

In Example 5, the subject matter of Examples 1-4 optionally includes, wherein the first ramp is obliquely angled relative to the second ramp, wherein the first ramp is configured to pass a contact head of coupling assembly into a position partially within or adjacent a notch of the trunnion, and wherein the second ramp is configured to bring the contact head into the engagement with the trunnion within the notch.

In Example 6, the subject matter of Examples 1-5 includes, wherein the second slot is at least partially formed by a rail, wherein together the first ramp, the second ramp and the rail act to guide translation of the coupling assembly within respective ones of the first slot and second slot.

In Example 7, the subject matter of Example 6 optionally includes, wherein the first ramp, the second ramp and the rail are configured to offload at least some of a coupling force from the engagement of the coupling assembly with the trunnion to the frame to thereby reduce the coupling force on the actuation assembly.

In Example 8, the subject matter of Examples 1-7 optionally includes, wherein the frame forms a distal end of the second slot and engagement with the distal end limits travel of the coupling assembly along the first slot.

In Example 9, the subject matter of Examples 1-8 optionally includes, wherein the frame is configured as a static cam and the coupling assembly is configured as a follower guided by the static cam.

In Example 10, the subject matter of Examples 1-9 optionally includes, wherein coupling assembly includes a first post, a second post and a linkage member extending between the first post and the second post, wherein the first post is received in the first slot, the second post is received in the second slot and the linkage member at least partially receives a distal end portion of the actuation assembly.

In Example 11, the subject matter of Examples 1-10 optionally includes, wherein the proximal end portion of the frame is configured to be coupled with a power driver.

Example 12 is a handle adapter for manipulating an orthopedic tool, optionally comprising: a frame and having a proximal end portion and a distal end portion, the distal end portion including: a receptacle configured to receive a trunnion of the orthopedic tool; a first slot communicating with the receptacle, wherein the first slot is partially formed by a first ramp and a second ramp; and a second slot spaced from the first slot and at least partially formed by a rail; a coupling assembly received by the first slot and the second slot; and an actuation assembly configured to drivingly engage the coupling assembly into engagement with the trunnion, wherein the first ramp, the second ramp and the rail are configured to offload at least some of a coupling force from the engagement of the coupling assembly with the trunnion to the frame to thereby reduce the coupling force on the actuation assembly.

In Example 13, the subject matter of Example 12 optionally includes, wherein the proximal end portion of the frame is configured to be coupled with a power driver.

In Example 14, the subject matter of Examples 12-13 optionally includes, wherein coupling assembly includes a first post, a second post and a linkage member extending between the first post and the second post, wherein the first post is received in the first slot, the second post is received in the second slot and the linkage member at least partially receives a distal end portion of the actuation assembly.

In Example 15, the subject matter of Examples 12-14 optionally includes, wherein the second slot is configured to limit travel of the coupling assembly along the first slot.

In Example 16, the subject matter of Examples 12-15 optionally includes, wherein together the first ramp, the second ramp and the rail act to guide translation of the coupling assembly within respective ones of the first slot and second slot, and wherein the first ramp is configured to pass a contact head of coupling assembly into a position adjacent a notch of the trunnion, and wherein the second ramp is configured to bring the contact head into the engagement with the trunnion within the notch.

Example 17 is a handle adapter for manipulating an orthopedic tool, optionally comprising: a frame and having a proximal end portion and a distal end portion, the distal end portion including: a receptacle configured to receive a trunnion of the orthopedic tool; a first slot communicating with the receptacle, wherein the first slot is partially formed by a first ramp and a second ramp; and a second slot spaced from the first slot; a coupling assembly received by the first slot and the second slot; and an actuation assembly configured to drivingly engage the coupling assembly into engagement with the trunnion, wherein the first ramp is configured to pass a contact head of coupling assembly into a position adjacent a notch of the trunnion, and wherein the second ramp is configured to bring the contact head into the engagement with the trunnion within the notch.

In Example 18, the subject matter of Example 17 optionally includes, wherein the second slot is at least partially formed by a rail, wherein together the first ramp, the second ramp and the rail act to guide translation of the coupling assembly within respective ones of the first slot and second slot.

In Example 19, the subject matter of Examples 17-18 optionally includes, wherein the first ramp is configured to pass a contact head of coupling assembly into a position adjacent a notch of the trunnion, and wherein the second ramp is configured to bring the contact head into the engagement with the trunnion within the notch.

Example 20 is a method of coupling an orthopedic tool to a handle adapter, optionally comprising: positioning a frame of the handle adapter to receive a trunnion of the orthopedic tool therein; translating a coupling assembly within a first slot and a second slot of the frame including along a first ramp and a second ramp of the first slot; and engaging the coupling assembly with the trunnion with the coupling assembly positioned on the second ramp.

Example 21 is at least one machine-readable medium including instructions that, when executed by processing circuitry, cause the processing circuitry to perform operations to implement of any of Examples 1-20.

Example 22 is an apparatus comprising means to implement of any of Examples 1-20.

Example 23 is a system to implement of any of Examples 1-20.

Example 24 is a method to implement of any of Examples 1-20.

In Example 25, is the handle adapter, machine-readable medium, system or method of any one or any combination of Examples 1-24 can optionally be configured such that all elements or options recited are available to use or select from.

### BRIEF DESCRIPTION OF THE DRAWINGS

In the drawings, like numerals can be used to describe similar elements throughout the several views. Like numerals having different letter suffixes can be used to represent different views or features of similar elements. The drawings illustrate generally, by way of example, but not by way of limitation, various embodiments discussed in the present document.
FIG. 1 is a cross-sectional view of a handle adapter coupled with a broach in accordance with at least one example of the present disclosure.
FIG. 2 is a perspective view of the handle adapter of FIG. 1 with some internal components thereof shown in phantom in accordance with at least one example of the present disclosure.
FIG. 3 is a perspective view of a coupling assembly including a post and a linkage member in accordance with at least one example of the present disclosure.
FIG. 4 is a side view of the handle adapter with portions an actuation assembly and the coupling assembly shown in phantom in accordance with at least one example of the present disclosure.
FIGS. 5A, 5B and 5C shows linear translational movement of the coupling assembly with respect to a trunnion of the orthopedic tool and a frame of the handle adapter from a disengaged position of FIG. 5A to an engaged position of FIG. 5C in accordance with at least one example of the present disclosure.
FIG. 5D shows linear translational movement of the coupling assembly with respect to the frame of the handle adapter to a lever locked position with the orthopedic tool removed in accordance with at least one example of the present disclosure.
FIGS. 6A and 6B show a distal end portion of another example of the handle adapter with the coupling assembly thereof engaging another example of the trunnion of a second orthopedic tool in accordance with at least one example of the present disclosure.
FIG. 7A, 7B, 7C, 7D, and 7E show actuation of components of the handle adapter of FIGS. 6A and 6B including operation of a locking mechanism in accordance with at least one example of the present disclosure.

Corresponding reference characters indicate corresponding parts throughout the several views. The exemplifications set out herein illustrate exemplary embodiments of the disclosure, and such exemplifications are not to be construed as limiting the scope of the disclosure in any manner.

### DETAILED DESCRIPTION

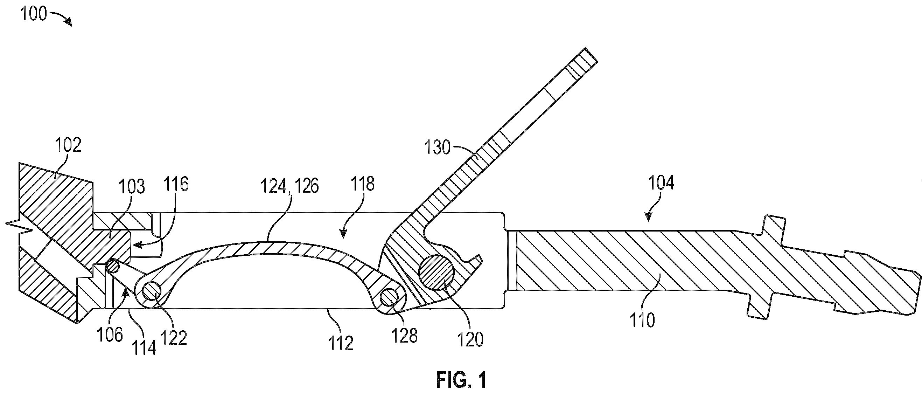

FIGS. 1-7E illustrate various examples of a handle adapter for releasably coupling with and manipulating an orthopedic cutting tool, such as a broach. Although examples described herein are shown coupled with and decoupled from a broach, the present application contemplates that other tools can be used with the handle adapter examples of the present application. These orthopedic tools can include, but are not limited to, rasps, awls, reamers, angled drivers, twist drills, flexible drills, cannulated drills, bayonet drills, bayonet taps, drill guides, adjustable angle drill guides, taps, and cannulated taps.

FIG. 1 shows a cross-sectional view of a handle adapter 100 according to one example. The handle adapter 100 is shown coupled to an orthopedic tool 102 (also shown in cross-section) having a trunnion 103. The handle adapter 100 can include a frame 104, a coupling assembly 106 and an actuation assembly 108. The frame 104 can include a proximal end portion 110, an intermediate portion 112, a distal end portion 114, a receptacle 116 and a recess 118.

FIG. 1 shows the handle adapter 100 in coupling engagement with the orthopedic tool 102 via the coupling assembly 106. More particularly, the frame 104 can be configured to receive the trunnion 103 of the orthopedic tool 102 via the receptacle 116 at the distal end portion 114. The trunnion 103, when received in the receptacle 116, can be selectively engaged by and held in position with the coupling assembly 106 as driven by the actuation assembly 108.

As shown in FIG. 1, the frame 104 has an elongate length extending from the proximal end portion 110, the intermediate portion 112 to the distal end portion 114. The proximal end portion 110 can be configured to couple with a driver (not shown) such as a powered tool that can be used to vibrate or otherwise move the handle adapter 100 and the orthopedic tool 102 as known in the art. According to other examples, the proximal end portion can be configured for manual manipulation such as by having a strike plate or other feature. The frame 104 can have a single piece construction with the proximal end portion 110 integrally formed with the intermediate portion 112 and the distal end portion 114.

The intermediate portion 112 can include one or more walls (not shown) enclosing the recess 118. The recess 118 can extend to the distal end portion 114. The recess 118 can house components including parts of the coupling assembly 106 and the actuation assembly 108. The actuation assembly 108 can be moveably connected to the intermediate portion 112 of the frame 104 by a first pin 120 about which portions of the actuation assembly 108 such as a lever can pivot. The coupling assembly 106 can be coupled to the actuation assembly 108 such as via a second pin 122. The second pin 122 can be constrained by engagement with the frame 104 to facilitate linear movement of the actuation assembly 108 and the coupling assembly 106 relative to the frame 104 as further discussed herein. The coupling assembly 106 can engage the distal end portion 114 of the frame 104 and can have a guided and controlled movement relative thereto as further discussed herein. In particular, the frame 104 can be configured as a static or stationary cam and the coupling assembly 106 is configured as a follower (as driven by the actuation assembly 108) guided by the static cam (the frame 104) into the coupling engagement with the orthopedic tool 102 as shown in FIG. 1.

The actuation assembly 108 can include a member 124 such as a rod, linkage bar, shaft or spring 126, a third pin 128 and a lever 130. The member 124 can be received in the recess 118 and can couple to the coupling assembly 106 at a distal end thereof. The third pin 128 can be received in the recess 118 at a proximal end of the member 124. The member 124 can be coupled to the lever 130 by the third pin 128. The lever 130 can be coupled to the frame 104 for pivotal movement by the first pin 120. Thus, the first pin 120 can comprise a hinge pin, for example. The lever 130 can extend from the recess 118 external of the frame 104. The lever 130 can be configured as a handle for grasping and actuating a movement of the member 124 relative to the frame 104.

The member 124 can be a linkage between the lever 130 and the coupling assembly 106. The member 124 can be an elastic component that is deflectable during operable use. Thus, the member 124 can be configured as the spring 126. The spring 126 can have a generally curved or arcuate portion between the coupling ends that receive the second pin 122 and the third pin 128, respectively. The spring 126 can be positioned such that the curved or arcuate portion is within the recess 118. The spring 126 can be subject to a compressive load when the coupling assembly 106 is in the engagement with the trunnion 103.

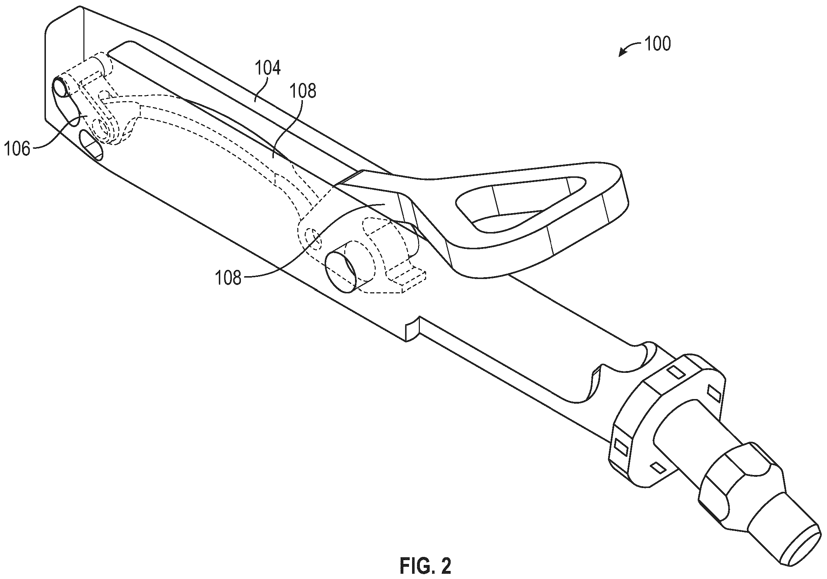

FIG. 2 shows a perspective view of the handle adapter 100 including the frame 104 with the coupling assembly 106 and parts of the actuation assembly 108 including the member 124 shown in phantom. The orthopedic tool 102 is not shown in FIG. 2.

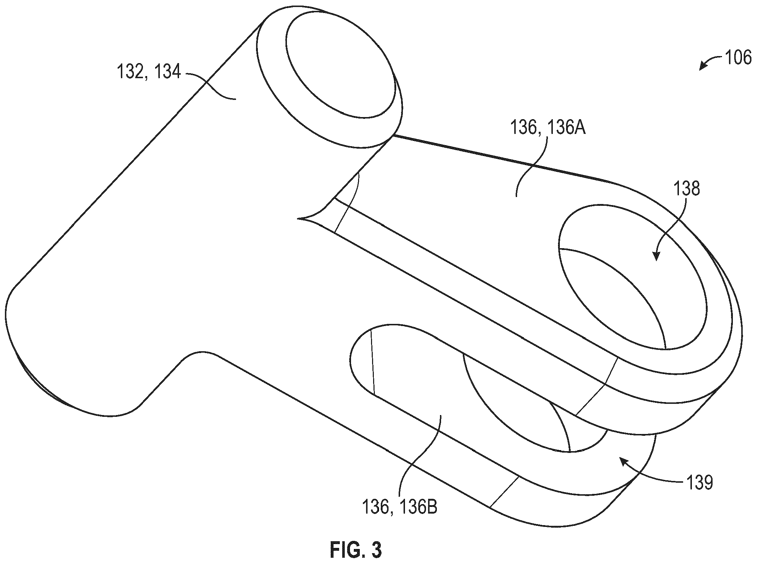

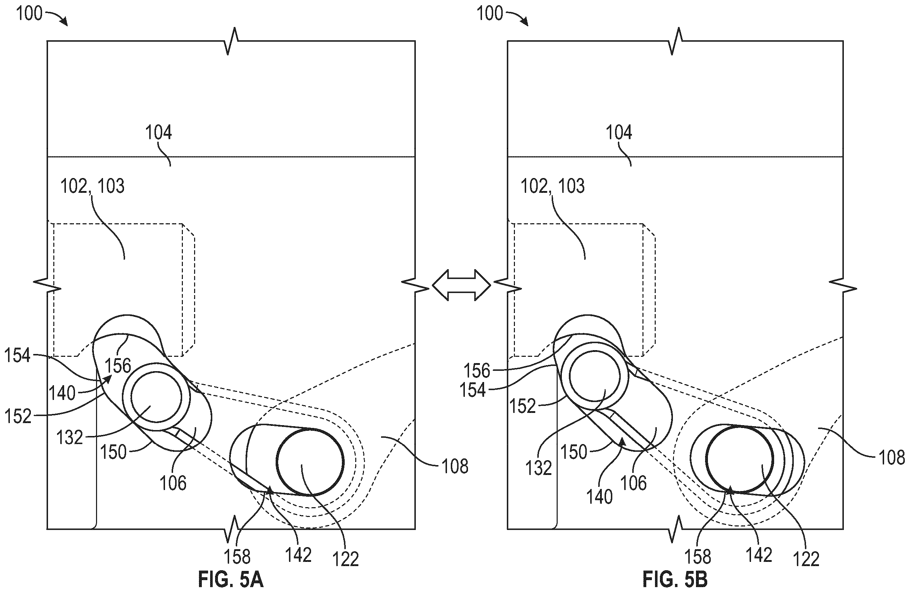

FIG. 3 is a perspective view of the coupling assembly 106 according to one example. The coupling assembly 106 can include a first contact head 132 configured as a first post or projection 134, a linkage member 136 and coupling receptacle 138. The first contact head 132 can project laterally from the linkage member 136 on opposing sides thereof. The linkage member 136 can have arms 136A and 136B connected by the first contact head 132. The linkage member 136 can be configured as an open frame having the arms 136A and 136B extending generally longitudinally in a spaced relationship. The arms 136A and 136B can be spaced apart from one another to create a recess 139 configured to receive a distal end of the member 124 (not shown). The arms 136A and 136B at proximal end portions thereof can form the coupling receptacle 138. The coupling receptacle 138 can be configured to receive the second pin 122 (FIG. 1) therein as further discussed and illustrated with regards to FIGS. 5A-5C, for example.

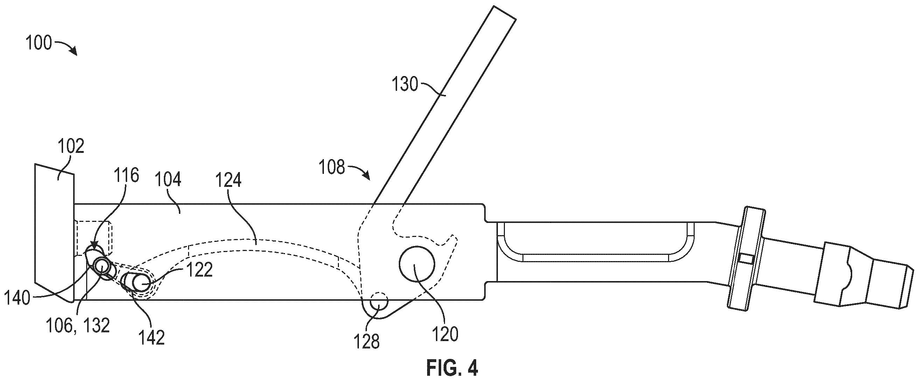

For the coupling assembly 106, the first contact head 132 can comprise a first post or pin, for example. The second pin 122 (FIG. 1) can act as a second post. The linkage member 136 can extend between the first post (first contact head 132) and the second post (the second pin 122 see FIG. 1). Thus, the first post can be received in a first slot, the second post can be received in a second slot and the linkage member 136 can at least partially receives a distal end portion of the actuation assembly 108 as further discuss below in regard to FIGS. 4-5C.

FIG. 4 shows the handle adapter 100 in a decoupled position relative to the orthopedic tool 102. FIG. 4 is a side view of the handle adapter 100 with the lever 130 raised away at an angle from the frame 104 and components of the actuation assembly 108 and the coupling assembly 106 shown in phantom moved away from engagement with the orthopedic tool 102. FIG. 4 additionally shows some of the components discussed previously including the first pin 120, the second pin 122, the member 124, the third pin 128. Additionally, FIG. 4 illustrates the frame 104 at the distal end portion 114 can include a first slot 140 and a second slot 142. The first slot 140 can communicate with the receptacle 116 and is configured to receive portions of the coupling assembly 106 such as the first contact head 132. The second slot 142 is configured to receive the second pin 122 therein. Together, the first slot 140 and the second slot 142 can guide linear movement of the coupling assembly 106 into locking engagement with the orthopedic tool 102 and out of locking engagement therewith. Additionally, the frame 104 that forms the first slot 140 and the second slot 142 can act to offload at least some of a extraction force from the engagement of the coupling assembly 106 with the orthopedic tool 102 to the frame 104 to thereby reduce a reaction force on the actuation assembly 108 that results from the tension between the coupling assembly 106 and the orthopedic tool 102.

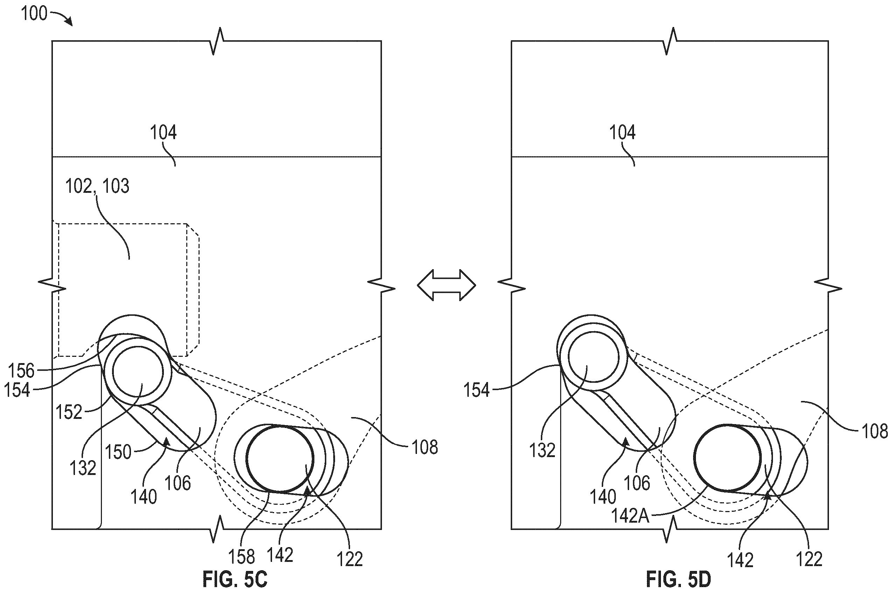

FIGS. 5A-5C illustrate an example of linear translational movement of the coupling assembly 106 with respect to the trunnion 103 of the orthopedic tool 102 and with respect to the frame 104 of the handle adapter 100 from the disengaged position of FIG. 5A (also shown previously in FIG. 4) to an engaged position of FIG. 5C.

FIGS. 5A-5C show a side view of the handle adapter 100 with portions of components of the actuation assembly 108 and the coupling assembly 106 shown in phantom as they would be covered by the frame 104. FIGS. 5A-5C show the distal end portion 114 with the first slot 140 and the second slot 142. The first slot 140 can receive the first contact head 132 and the second slot 142 can receive the second pin 122 therein.

As shown in FIGS. 5A-5C, a portion of the frame 104 defines the first slot 140 and the frame 104 can have a first ramp 150, a transition 152 and a second ramp 154 defining portions of the first slot 140. The first ramp 150 and the second ramp 154 can form one or more edges of the first slot 140. The first ramp 150 can be angled (e.g., obliquely angled) relative to the second ramp 154. The transition 152 can be positioned between the first ramp 150 and the second ramp 154 and can be a point or area on the frame 104.

According to one example, the first ramp 150 can be configured (oriented, angled, positioned, sized) to allow the first contact head 132 to pass into a notch 156 of the trunnion 103. Put another way, the first ramp 150 can allow for at least a portion of the first contact head 132 to pass into the notch 156. Thus, the first ramp 150 can be configured to pass the first contact head 132 into a position partially within or adjacent the notch 156 of the trunnion 103. The transition 152 can be positioned opposite a point of first engagement between the first contact head 132 with the trunnion 103. The second ramp 154 can be configured (oriented, angled, positioned, sized) to allow the first contact head 132 to incrementally engage the trunnion 103 with increasing force as the first contact head 132 travels further along the second ramp 154 from the transition 152. Thus, the second ramp 154 can be configured to bring the first contact head 132 into engagement with the trunnion 103 within the notch 156.

FIGS. 5A-5C show the second slot 142, which can be spaced from the first slot 140 by the distal end portion 114 of the frame 104. The second slot 142 can be at least partially formed by a rail 158. The rail 158 can be formed by one or more edges of the frame 104 along the second slot 142. The rail 158 can be configured to proscribe linear movement of the actuation assembly 108 and the coupling assembly 106 as the second pin 122 is received in the second slot 142 and engages the rail 158. The second pin 122 can selectively travel along the second slot 142 with movement proscribed by the rail 158 as illustrated in FIGS. 5A-5D.

FIG. 5A shows the coupling assembly 106 moved to a disengaged position relative to the trunnion 103 of the orthopedic tool 102. The coupling assembly 106 is moved to a position having the first contact head 132 resting against the first ramp 150 and the second pin 122 is positioned at or adjacent a proximal end of the second slot 142 on the rail 158.

FIG. 5B shows the coupling assembly 106 moved to an initial engagement position relative to the trunnion 103 with the first contact head 132 engaging the trunnion 103 and resting on the transition 152. The second pin 122 has traveled distally along the second slot 142 on the rail 158.

FIG. 5C shows the coupling assembly 106 moved to a second engagement position relative to the trunnion 103 with the first contact head 132 engaging the trunnion 103 and resting on the second ramp 154. The second pin 122 as traveled even further distally to a position adjacent a distal end of the second slot 142 on the rail 158. As shown in FIGS. 5A-5C, together the first ramp 150, the second ramp 154 and the rail 158 guide translation of the coupling assembly 106 within the respective ones of the first slot 140 and the second slot 142.

FIG. 5D shows the trunnion 103 removed from being received by the frame 104 and the coupling assembly 106 and the actuation assembly 108 moved relative to the frame 104 to a locked position. Travel of the coupling assembly 106 and the actuation assembly 108 including the first contact head 132 along the first slot 140 can be limited by contact of the second pin 122 with a distal end 142A of the second slot 142. The first contact head 132 can remain on the second ramp 154. In the locked position, the lever (not shown) can be moved down to a position adjacent the frame 104 (e.g., a zero angle or near zero angle position).

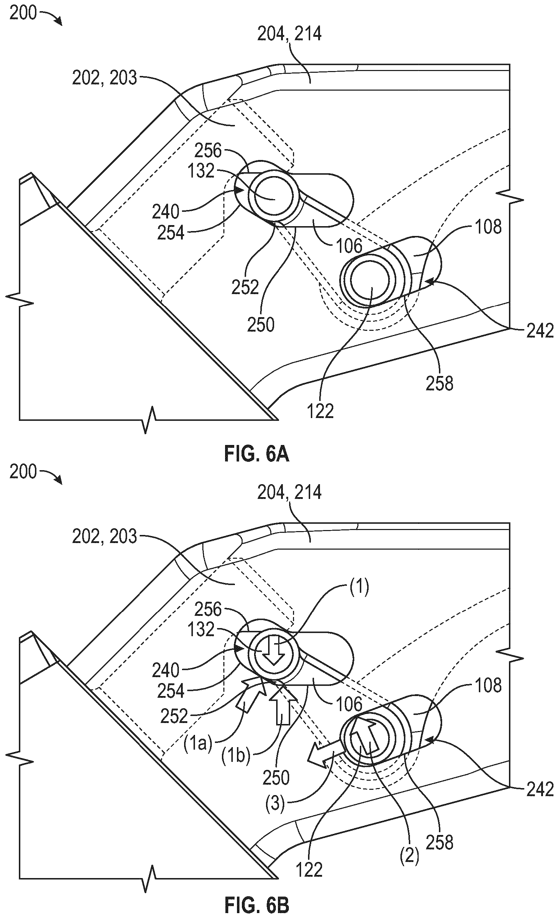

FIGS. 6A and 6B show another example of a handle adapter 200 engaging a trunnion 203 of an orthopedic tool 202. FIGS. 6A and 6B show a side view of the handle adapter 200 with portions of components of the actuation assembly 108 and the coupling assembly 106 shown in phantom as they would be covered by a frame 204. The handle adapter 200 can include components such as the actuation assembly 108 and the coupling assembly 106 similar to those discussed previously but with the frame 204 modified. The frame 204 is modified as the trunnion 203 differs in geometry including having a notch 256 with a different geometry than that of the trunnion 103 (FIGS. 5A-5C) discussed previously. The different geometry for the trunnion 203 can include a longer length and a different geometry for the notch 256, for example.

As shown in FIGS. 6A and 6B, the handle adapter 200 includes a distal end portion 214 with a first slot 240 and a second slot 242. The first slot 240 and the second slot 242 can be configured with a different geometry from the first slot 140 and the second slot 142 previously discussed. The first slot 240 can receive the first contact head 132 and the second slot 242 can receive the second pin 122.

The frame 204 defines the first slot 240 and the frame 204 can have a first ramp 250, a transition 252 and a second ramp 254 defining portions of the first slot 240. The first ramp 250 and the second ramp 254 can form one or more edges of the first slot 240. The first ramp 250 can be angled (e.g., obliquely angled) relative to the second ramp 254. The transition 252 can be positioned between the first ramp 250 and the second ramp 254 and can be a point or area on the frame 204.

The first ramp 250 can be configured (oriented, angled, positioned, sized) to allow the first contact head 132 to pass into the notch 256 of the trunnion 203. The first ramp 250 for example can be oriented substantially parallel with a surface of the trunnion 203 that forms part of the notch 256 at an opening thereto. The first ramp 250 can allow for at least a portion of the first contact head 132 to pass into the notch 256. Thus, the first ramp 250 can be configured to pass the first contact head 132 into a position partially within or adjacent the notch 256 of the trunnion 203. The transition 252 can be positioned opposite a point of first engagement between the first contact head 132 with the trunnion 203. The second ramp 254 can be configured (oriented, angled, positioned, sized) to allow the first contact head 132 to incrementally engage the trunnion 203 with increasing force as the first contact head 132 travels further along the second ramp 254 from the transition 252. Thus, the second ramp 254 can be configured to bring the first contact head 132 into engagement with the trunnion 203 within the notch 256.

As shown in FIGS. 6A and 6B, the second slot 242 can be at least partially formed by a rail 258. The rail 258 can be formed by one or more edges of the frame 204 along the second slot 242. The rail 258 can be configured to proscribe linear movement of the actuation assembly 108 and the coupling assembly 106 as the second pin 122 is received in the second slot 242 and engages the rail 258.

FIGS. 6A and 6B show the coupling assembly 106 in particular the first contact head 132 engaged with the trunnion 203. FIG. 6B shows a resolution of vectors for extraction forces that occur with such engagement during use of the orthopedic tool 202. FIG. 6B shows at least some extraction force resulting from the coupling of the first contact head 132 with the trunnion 203 is resolved to the frame 204 rather than the entire extraction force being passed to the actuation assembly 108, and in particular, the member 124. Arrow (1) shows a vector of the extraction force from the trunnion 203 to the first contact head 132. Arrow (1a) shows an immediate offloading of at least some of this extraction force to the frame 204 at the second ramp 254. Should the first contact head 132 slip down to the transition 252, arrow (1b) shows offloading remains to the frame 204 as the first contact head 132 remains in engagement with the trunnion 203. The force vector indicated by arrow (1b) can be generally aligned with the extraction force indicated by arrow (1). A force vector (indicated by arrow (3)) applied by the member 124 and the actuation assembly 108 can maintain the engagement between the first contact head 132 and the trunnion 203. Force vector (2) shows the second pin 122 and frame 204 react the extraction force on the coupling assembly 106 such that at least some of the extraction force is carried by the frame 204 rather than an entirety thereof being transmitted to the member 124 and the actuation assembly 108.

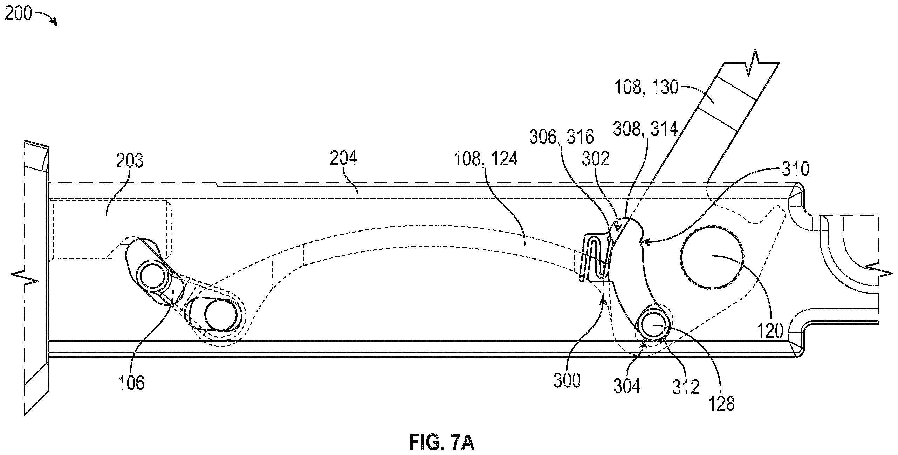

FIGS. 7A-7E show other aspects of the handle adapter 200, in particular, a locking mechanism 300. The locking mechanism 300 can be used to reduce vibration of the lever 130 when driven by a powered tool, can maintain the lever 130 in the locked position adjacent the frame 204 and/or can maintain the coupling assembly 106 in engagement with the trunnion 203. The frame 204 and the actuation assembly 108 can be modified with the locking mechanism 300.

The locking mechanism 300 can include a third slot 302, a fourth slot 304, a spring 306, a seat 308, a pin support 310 and the third pin 128. The locking mechanism 300 can be positioned at an intermediate or proximal portion of the frame 204 and can be positioned adjacent the recess 118 discussed previously. As shown in FIG. 7A, the locking mechanism 300 can be positioned to interact with the connection between the member 124 and the lever 130. FIG. 7A shows portions the coupling assembly 106 and the actuation assembly 108 in phantom.

As shown in FIG. 7A, the third slot 302 can be formed by the frame 204 and can extend generally laterally across a longitudinal axis LA in addition to have a longitudinal extent along the longitudinal axis LA. The third slot 302 can be arcuate in shape having a first end 312 and a second end 314. The first end 312 of the third slot 302 can receive the third pin 128 when the lever 130 is in the open (disengaged) position as shown in FIG. 7A. The third pin 128 can travel along the third slot 302 toward and to the second end 314 with closing of the lever 130 toward and to engagement position (not shown in FIG. 7A). The spring 306, seat 308 and the pin support 310 can be located adjacent the second end 314 of the third slot 302. The spring 306 can be formed from the frame 204 and can define a portion of the third slot 302, for example. However, other examples contemplate the spring 306 as a dedicated component separate from the frame 204.

The seat 308 can be formed by the frame 204 at the second end 314 of the third slot 302 and can additionally be formed by the pin support 310 at a second side. The pin support 310 can be a projection configured to receive a part of the third pin 128. The pin support 310 can be positioned opposite an engagement head 316 of the spring 306.

The fourth slot 304 can be formed by a distal portion of the lever 130, for example. The fourth slot can have an elongate length and can be configured to receive the third pin 128. The fourth slot 304 can be generally positioned adjacent the third slot 302 as both the third slot 302 and the fourth slot 304 receive the third pin 128. The fourth slot 304 allows the lever 130 to have some degree of play/movement with regard to the third pin 128. The third pin 128 can also be received by an aperture or slot of the member 124 as shown in FIG. 7A.

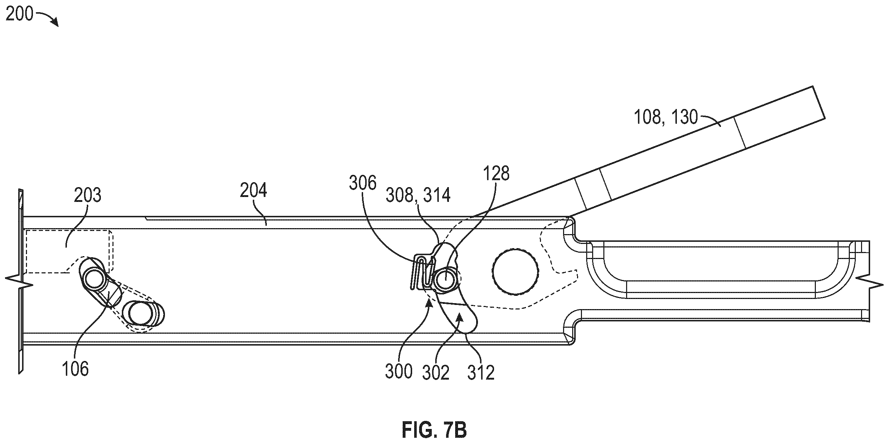

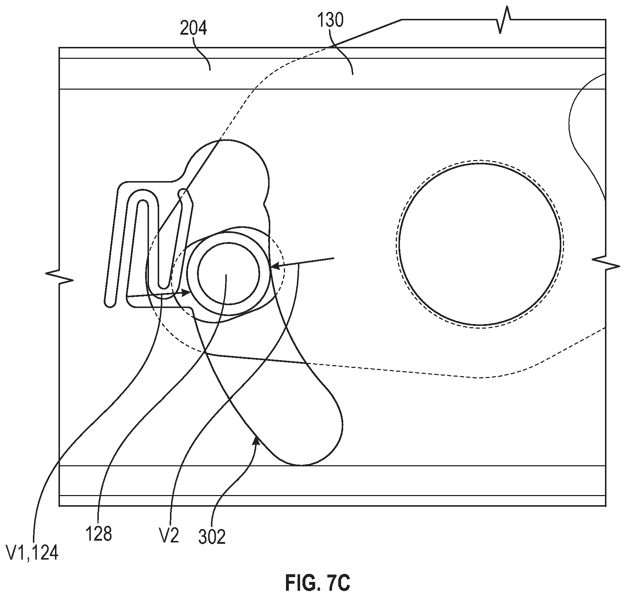

FIGS. 7B-7E show engaging movement of the actuation assembly and the coupling assembly from the disengaged position of FIG. 7A. FIG. 7B shows the first contact head 132 moved to make initial contact with the trunnion 203. The third pin 128 has traveled to an intermediate position along the third slot 302 between the first end 312 and the second end 314. FIG. 7C is an enlarged view from FIG. 7B that shows vector reproduction of the forces (V1 from spring 124 and V2 from the frame 204) on the third pin 128. The lever 130 does not see a force from the member 124 (see FIG. 7A). Rather the force from the member 124 (FIG. 7A) is resolved into the frame 204, in particular, the force is applied against a rail that forms an edge of the third slot 302. This arrangement for the forces can reduce wear on the lever 130 and lever pin 120 (FIG. 7A).

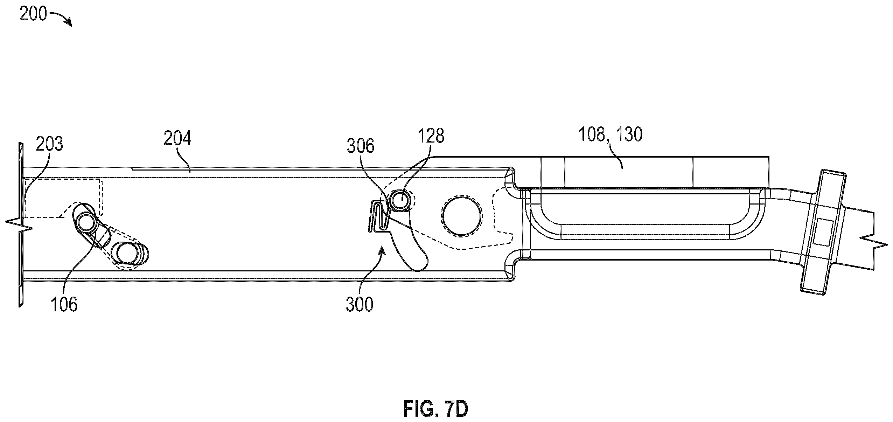

FIGS. 7D shows the actuation assembly 108 and the coupling assembly 106 moved to a fully engaged position with the coupling assembly 106 in contact with the trunnion 203. The lever 130 can be retained against or adjacent the frame 204 in a zero angle or near zero angle position as shown in FIG. 7D. The locking mechanism 300 acts to lock/retain the actuation assembly 108 and the coupling assembly 106 in the fully engaged position. Additionally, the locking mechanism 300 acts to inhibit or reduce vibration of the lever 130 relative to the frame 204 during powered driving operation.

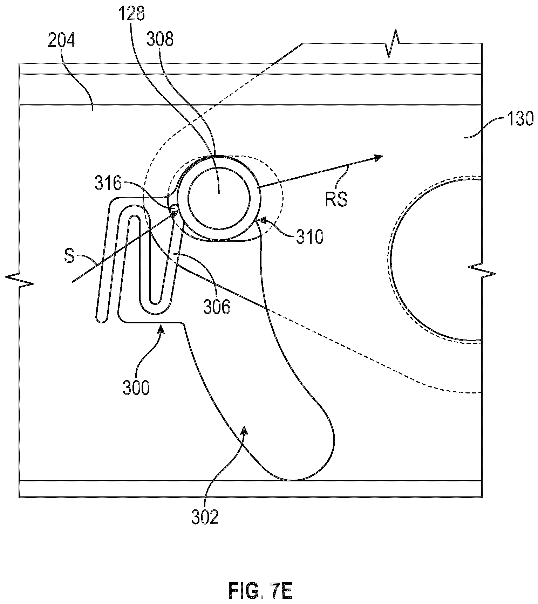

As shown in the enlarged view of FIG. 7E, in the fully engaged position with the locking mechanism 300 retaining the actuation assembly 108 and the coupling assembly 106, the third pin 128 can be received in the seat 308 and is engaged by the engagement head 316 of the spring 306 and the pin support 310. The spring 306 can force the third pin 128 into the seat 308. FIG. 7E shows a vector representation of forces acting on the third pin 128 in fully engaged position. The force (S) of the spring 306 on the third pin 128 is resolved as a force (RS) into the frame 204 as shown in FIG. 7E. The force (RS) is applied against the rail that forms the edge of the third slot 302 at the seat 308 rather than be applied to the lever 130. This reduces wear on the lever 130 and lever pin 120 (FIG. 7A) as discussed previously.

The above Detailed Description includes references to the accompanying drawings, which form a part of the Detailed Description. The drawings show, by way of illustration, specific embodiments in which the handle assembly and related methods can be practiced. These embodiments are also referred to herein as "examples." While certain examples are shown and described with respect to a left or a right handle orientation, it is to be appreciated that the present disclosure is equally applicable to both the left and right handles.

The above Detailed Description is intended to be illustrative, and not restrictive. For example, the above-described examples (or one or more elements thereof) can be used in combination with each other. Other embodiments can be used, such as by one of ordinary skill in the art upon reviewing the above description. Also, various features or elements can be grouped together. This should not be interpreted as intending that an unclaimed disclosed feature is essential to any claim. Rather, inventive subject matter can lie in less than all features of a particular disclosed embodiment. Thus, the following claims are hereby incorporated into the Detailed Description, with each claim standing on its own as a separate embodiment. The scope of the invention should be determined with reference to the appended claims, along with the full scope of equivalents to which such claims are entitled.

In this document, the terms "a" or "an" are used to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, "anterior" refers to a direction generally toward the front of a patient, "posterior" refers to a direction generally toward the back of the patient, "medial" refers to a direction generally toward the middle of the patient, and "lateral" refers to a direction generally toward the side of the patient. In this document, the phrase "anterior/posterior direction" is used to include an anterior to posterior direction or a posterior to anterior direction.

In the appended claims, the terms "generally", "substantially" and "about" mean within ± 15% of the example described. The terms "including" and "in which" are used as the plain-English equivalents of the respective terms "comprising" and "wherein." The terms "including" and "comprising" are open-ended, that is, an apparatus, system, kit, or method that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

## Claims

1. A handle adapter for manipulating an orthopedic tool, comprising:
a frame and having a proximal end portion and a distal end portion, the distal end portion including:
a receptacle configured to receive a trunnion of the orthopedic tool;
a first slot communicating with the receptacle, wherein the first slot is partially formed by a first ramp and a second ramp; and
a second slot spaced from the first slot;
a coupling assembly received by the first slot and the second slot, wherein the coupling assembly translates within the first slot and the second slot; and
an actuation assembly coupled to the coupling assembly and configured to drive the coupling assembly into engagement with the trunnion.

2. The handle adapter of claim 1, wherein the actuation assembly includes a spring coupled to a lever, and wherein the spring is coupled to and drives the coupling assembly into the engagement with the trunnion.

3. The handle adapter of claim 2, wherein the spring is subject to a compressive load when the coupling assembly is in the engagement with the trunnion.

4. The handle adapter of any one of claims 2-3, further comprising a locking mechanism that locks the handle in a closed position relative to the frame, wherein the locking mechanism includes a spring formed by the frame.

5. The handle adapter of any one of claims 1-4, wherein the first ramp is obliquely angled relative to the second ramp, wherein the first ramp is configured to pass a contact head of coupling assembly into a position partially within or adjacent a notch of the trunnion, and wherein the second ramp is configured to bring the contact head into the engagement with the trunnion within the notch.

6. The handle adapter of any one of claims 1-5, wherein the second slot is at least partially formed by a rail, wherein together the first ramp, the second ramp and the rail act to guide translation of the coupling assembly within respective ones of the first slot and second slot.

7. The handle adapter of claim 6, wherein the first ramp, the second ramp and the rail are configured to offload at least some of a coupling force from the engagement of the coupling assembly with the trunnion to the frame to thereby reduce the coupling force on the actuation assembly.

8. The handle adapter of any one of claims 1-7, wherein the frame forms a distal end of the second slot and engagement with the distal end limits travel of the coupling assembly along the first slot.

9. The handle adapter of any one of claims 1-8, wherein the frame is configured as a static cam and the coupling assembly is configured as a follower guided by the static cam.

10. The handle adapter of any one of claims 1-9, wherein coupling assembly includes a first post, a second post and a linkage member extending between the first post and the second post, wherein the first post is received in the first slot, the second post is received in the second slot and the linkage member at least partially receives a distal end portion of the actuation assembly.

11. The handle adapter of any one of claims 1-10, wherein the proximal end portion of the frame is configured to be coupled with a power driver.

12. A handle adapter for manipulating an orthopedic tool, comprising:
a frame and having a proximal end portion and a distal end portion, the distal end portion including:
a receptacle configured to receive a trunnion of the orthopedic tool;
a first slot communicating with the receptacle, wherein the first slot is partially formed by a first ramp and a second ramp; and
a second slot spaced from the first slot and at least partially formed by a rail;
a coupling assembly received by the first slot and the second slot; and
an actuation assembly configured to drivingly engage the coupling assembly into engagement with the trunnion, wherein the first ramp, the second ramp and the rail are configured to offload at least some of a coupling force from the engagement of the coupling assembly with the trunnion to the frame to thereby reduce the coupling force on the actuation assembly.

13. The handle adapter of claim 12, wherein the proximal end portion of the frame is configured to be coupled with a power driver.

14. The handle adapter of any one of claims 12-13, wherein coupling assembly includes a first post, a second post and a linkage member extending between the first post and the second post, wherein the first post is received in the first slot, the second post is received in the second slot and the linkage member at least partially receives a distal end portion of the actuation assembly.

15. A method of coupling an orthopedic tool to a handle adapter, comprising:
positioning a frame of the handle adapter to receive a trunnion of the orthopedic tool therein;
translating a coupling assembly within a first slot and a second slot of the frame including along a first ramp and a second ramp of the first slot; and
engaging the coupling assembly with the trunnion with the coupling assembly positioned on the second ramp.
